# EUROPEAN PATENT SPECIFICATION

(11) **EP 2 262 913 B1**
(45) Date of publication and mention of the grant of the patent: **13.02.2013**
(21) Application number: 09713394.6
(22) Date of filing: 19.02.2009
(51) Int. Cl.: C12Q 1/68

(54) **SYSTEMS AND METHODS OF CANCER STAGING AND TREATMENT**
SYSTEME UND VERFAHREN ZUR EINSTUFUNG UND BEHANDLUNG VON KREBS
SYSTÈMES ET PROCÉDÉS DE STADIFICATION ET DE TRAITEMENT DU CANCER

(30) Priority: 19.02.2008 US 29656 P
(43) Date of publication of application: 22.12.2010
(73) Proprietor: Translational Genomics Research Institute, Phoenix, AZ 85004 (US)
(72) Inventor: WEISS, Glen, Phoenix, AZ 85016 (US)
(74) Representative: Atkinson, Jennifer
(86) International application number: PCT/US2009/001046
(87) International publication number: WO 2009/105223

(56) References cited:
- US-A1- 2007 191 273
- US-A1- 2007 203 333
- FLAVIN R J ET AL: "Down-regulation of MIR-195 and MIR-497 from the MicroRNA cluster at chromosome 17p13.1 in papillary serous carcinoma of the peritoneum" MODERN PATHOLOGY, vol. 21, no. Suppl. 1, January 2008 (2008-01), page 204A, XP008108071 & 97TH ANNUAL MEETING OF THE UNITED-STATES-AND-CANADIAN-ACADEMY-OF-PATH OLOGY; DENVER, CO, USA; MARCH 01 -07, 2008 ISSN: 0893-3952
- CALIN G A ET AL: "Frequent deletions and down-regulation of micro-RNA genes miR15 and miR16 at 13q14 in chronic lymphocytic leukemia" PROCEEDINGS OF THE NATIONAL ACADEMY OF SCIENCES OF USA, NATIONAL ACADEMY OF SCIENCE, WASHINGTON, DC., US, vol. 99, no. 24, 26 November 2002 (2002-11-26), pages 15524-15529, XP002982123 ISSN: 0027-8424
- DATABASE EMBL [Online] 18 May 2007 (2007-05-18), "Sequence 1508 from Patent EP1777301." XP002535510 retrieved from EBI accession no. EMBL:CS548478 Database accession no. CS548478
- GU ET AL: "Identification and characterization of microRNAs from the bovine adipose tissue and mammary gland" FEBS LETTERS, ELSEVIER, AMSTERDAM, NL, vol. 581, no. 5, 24 February 2007 (2007-02-24), pages 981-988, XP005904577 ISSN: 0014-5793

## Description

### RELATED APPLICATIONS

This application claims priority to U.S. Provisional Application 61/029,656, filed 19 February 2008.

### FIELD

Provided herein are methods of treatment of cancer and more specifically methods of the differential treatment of cancer based upon microRNA gene expression.

### BACKGROUND

Worldwide, lung cancer is the leading cause of cancer-related mortality in both men and women. Although current treatments for advanced non-small cell lung cancer (NSCLC) are disappointing, there is growing promise using anti-angiogenic therapies. Some of these anti-angiogenic therapies, which include tyrosine kinase inhibitors (TKI) targeting VEGF receptor 2 and 3 (VEGFR-2/3), such as sunitinib and sorafenib, have been administered to patients in an unselected fashion.

MicroRNAs (also known as miRs or miRNAs) are a class of small non-coding RNAs having 21 to 25 nucleotides in length that have recently been implicated in cancer biology (Calin et al., Proc Natl Acad Sci USA 99:15524-15529, 2002). These RNA fragments post-transcriptionally regulate gene expression by binding to complementary sequences in the 3' untranslated region (3'UTR) of the target mRNA (Kumar et al., Nat Genet 39:673-677, 2007). This can ultimately lead to repression of protein translation and, as a result, of protein expression (Eder et al., N Eng J Med 352:2446-2448, 2005). Regulation of protein expression by miRs is emerging as an important area of study in carcinogenesis because their regulatory capabilities can drastically influence cell physiology (Scott et al., J Biol Chem 282:1479-1486, 2007). Therefore, there is a need for methods of predicting drug efficacy in individual patients by miR expression.
Flavin et al., Modern Pathology, 2008 vol 21, no. Suppl.1, page 204A discloses the down-regulation of MIR-195 and MIR-497 from the MicroRNA cluster at chromosome 17p13.1 in papillary serious carcinoma of the peritoneum.
US 2007/203333 A1 discloses RNA interference mediated inhibition of vascular endothelial growth factor and vascular endothelial growth factor receptor gene expression using short interfering nucleic acid (siNA)

### BRIEF SUMMARY

In one aspect, provided is a method of evaluating the sensitivity of non-small cell lung cancer cell to a tyrosine kinase inhibitor using microRNA.

In one embodiment, provided is a method of evaluating a cancer cell sensitivity to a tyrosine kinase inhibitor by assessing the expression of miR-497 in a patient sample and correlating a reduced expression of the miR-497 with a sensitivity to the tyrosine kinase inhibitor.

The method also provides a method of predicting a cancer cell sensitivity to a tyrosine kinase inhibitor in order to apply a personalized medicine based therapy to cancer treatment.

The method also provides is a method of predicting a cancer cell sensitivity to a tyrosine kinased inhibitor in order to prevent a patent from suffering side effects of a drug that is unlikely to be efficacious.

The method also provides is a method of predicting a cancer cell sensitivity to a tyrosine kinase inhibitor in order to prevent a cancer patient from wasting time on a treatment that is unlikely to be efficacious.

In one embodiment, a patient sample comes from blood. In another embodiment, a patient sample comes from a tumor biopsy.

In another embodiment, an expression is assessed by mRNA detection methods such as RTPCR, microarray analysis, or Northern blot.

In another embodiment, a tyrosine kinase inhibitor specifically inhibits VEGFR2 alone, VEGFR3 alone, both VEGFR2 and VEGFR3, or either VEGFR2 or VEGFR3 in combination with any other molecule. In one embodiment, a tyrosine kinase inhibitor is sunitinib. In another embodiment, a tyrosine kinase inhibitor is sorafenib.

In another aspect, provided is a method involving slowing the expansion of a non-small cell lung cancer cell including assessing the expression of miR-497 in a sample, correlating reduced expression of the miR-497 with sensitivity to a tyrosine kinase inhibitor, and treating a non-small cell lung cancer cell with the tyrosine kinase inhibitor

In one embodiment, a sample is taken from a human. In another embodiment, the sample is a blood fraction. In yet nother embodiment, the sample is a tumor biopsy.

In one embodiment, cancer cells are shown to display a loss of heterozygosity in chromosomal region 17p.

In one embodiment, a tyrosine kinase inhibitor specifically inhibits VEGFR2 alone, VEGFR3 alone, both VEGFR2 and VEGFR3, or either VEGFR2 or VEGFR3 in combination with any other molecule.

In another embodiment, a tyrosine kinase inhibitor is sunitinib.

### BRIEF DESCRIPTION OF THE DRAWINGS

A more complete understanding of the present invention may be derived by referring to the detailed description when considered in connection with the following illustrative figures. In the figures, like reference numbers refer to like elements or acts throughout the figures.

**Figure 1** depicts a Western blot image showing expression of VEGFR2 and VEGFR3 in H358 cells transfected with miR-497 mimic or control mimic microRNA.

**Figure 2** depicts the results of densitometry of Western blots showing expression of VEGFR2 and VEGFR3 in H358 cells transfected with miR-497 inhibitor or mimic or control inhibitor or mimic microRNA.

**Figure 3** depicts the results of densitometry of Western blots showing expression of VEGFR2 in H1703 cells transfected with miR-497 inhibitor or mimic or control inhibitor or mimic microRNA.

**Figure 4** depicts the results of densitometry of Western blots showing expression of VEGFR2 in H520 cells transfected with miR-497 inhibitor or mimic or control inhibitor or mimic microRNA.

**Figure 5** depicts the results of densitometry of Western blots showing expression of VEGFR2 in H157 cells transfected with miR-497 inhibitor or mimic or control inhibitor or mimic microRNA.

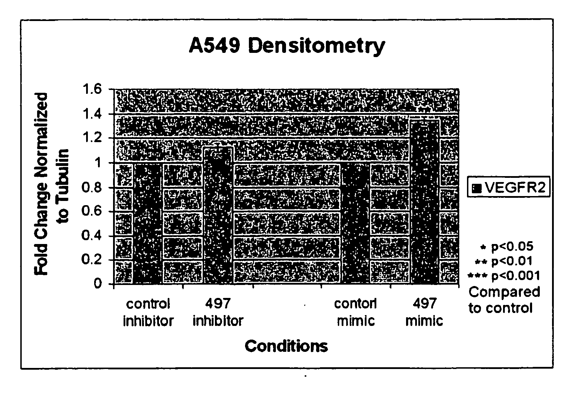

**Figure 6** depicts the results of densitometry of Western blots showing expression of VEGFR2 in H 1703 cells transfected with miR-497 inhibitor or mimic or control inhibitor or mimic microRNA.

**Figure 7** depicts the results of densitometry of Western blots showing expression of VEGFR2 and VEGFR3 in H1703 cells transfected with miR-497 inhibitor or mimic or control inhibitor or mimic microRNA.

**Figure 8** depicts the results of sunitinib treatment on the viability of sunitinib sensitive (H520 and H1703) and sunitinib resistant (H322c, H358, H157, and A549) cell lines.

**Figure 9** depicts the results ofqRT-PCR analysis of FGF1, LHFP, and HOXC10 in sunitinib sensitive (H520 and H1703) and sunitinib resistant (H322c, H358, H157, and A549) cell lines.

### DETAILED DESCRIPTION

### Definitions

Unless specifically noted, it is intended that the words and phrases in the specification and the claims be given their plain, ordinary, and accustomed meaning to those of ordinary skill in the applicable arts.

In the following description, and for the purposes of explanation, numerous specific details are set forth in order to provide a thorough understanding of the various aspects of the invention. It will be understood, however, by those skilled in the relevant arts, that the present invention may be practiced without these specific details. In other instances, known structures and devices are shown or discussed more generally in order to avoid obscuring the invention. The full scope of the disclosure is not limited to the examples that are described below.

### Methods of Use

Provided are methods of assessing the sensitivity of cancer cells to a drug using an miR-497 mimic in combination with a tyrosine kinase inhibitor. Also provided are methods of treating cancer with a tyrosine kinase inhibitor based upon the expression of miR-497 by the cancer cells.

A target includes any molecular structure produced by a cell and expressed inside the cell, on the cell surface, or secreted by the cell. Targets include proteins, lipids, carbohydrates, nucleic acids, including RNA molecules and genomic DNA sequences, subcellular structures, glycoproteins, viruses and any other like structures known or yet to be disclosed whether alone or in combination. Illustrative examples of targets include, but are not limited to, VEGFR2, VEGFR3, miR-497, FGF1, HOXC10, LHFP and any products thereof including mRNA's and proteins.

Cancer cells include any cells derived from a tumor, neoplasm, cancer, precancer, cell line, or any other source of cells that have the potential to expand and grow to an unlimited degree. Cancer cells are derived from naturally occurring sources or are artificially created. Cancer cells are capable of invasion into other tissues and metastasis when placed into an animal host. Cancer cells further encompass any malignant cells that have invaded other tissues and/or metastasized. One or more cancer cells in the context of an organism may also be called a cancer, tumor, neoplasm, growth, malignancy, or any other term used in the art to describe cells in a cancerous state.

Cancers that serve as sources of cancer cells include, but are not limited to, solid tumors such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

Additional cancers that serve as sources of cancer cells include, but are not limited to, blood borne cancers such as acute lymphoblastic leukemia, acute lymphoblastic B-cell leukemia, acute lymphoblastic T-cell leukemia, acute myeloblastic leukemia ,acute promyelocytic leukemia, acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia, chronic lymphocytic leukemia, hairy cell leukemia, multiple myeloma, lymphoblastic leukemia, myelogenous leukemia, lymphocytic leukemia, myelocytic leukemia, Hodgkin's disease, non-Hodgkin's lymphoma, Waldenstrom's macroglobulinemia, heavy chain disease, and polycythemia vera.

Non-small cell lung cancer (NSCLC) includes any carcinoma derived from lung tissues that does not include small cell lung cancers. Examples of non-small cell lung cancers include, but are not limited to, adenocarcenomas, large cell carcinomas, and squamous cell carcinomas of the lung.

Expansion of a cancer cell includes any process that results in an increase in the number of individual cells derived from a cancer cell. Expansion of a cancer cell may result from mitotic division, proliferation, or any other form of expansion of a cancer cell, whether in vitro or in vivo. Expansion of a cancer cell further encompasses invasion and metastasis. A cancer cell may be in physical proximity to cancer cells from the same clone or from different clones that may or may not be genetically identical to it. Such aggregations may take the form of a colony, tumor or metastasis, any of which may occur *in vivo* or *in vitro.* Slowing the expansion of the cancer cell may be brought about either by inhibiting cellular processes that promote expansion or by bringing about cellular processes that inhibit expansion. Processes that inhibit expansion include processes that slow mitotic division and processes that promote cell senescence or cell death. Examples of specific processes that inhibit expansion include caspase dependent and independent pathways, autophagy, necrosis, apoptosis, and mitochondrial dependent and independent processes and further include any such processes yet to be disclosed.

Inhibition of the expansion of a cancer cell is achieved through the use of an outside agent applied to a cancer cell for the purpose of slowing the expansion of a cancer cell. Such agents include natural or synthetic ligands, blockers, agonists, antagonists or activators of receptors, immune cells, such as CD8+ T cells, viruses, inhibitors of gene or protein expression, such as siRNA or miR's, small molecules, pharmaceutical compositions, or any other composition of matter that when administered to a cancer cell results in slowing of the expansion of a cancer cell. The concept of agents that slow the expansion of a cancer cell encompasses restricting access to any natural or artificial agent necessary for cell survival including necessary nutrients, ligands, or cell-cell contacts. Examples of such agents and conditions include treatment with antiangiogenic inhibitors.

In one embodiment, an agent that slows the expansion of a cancer cell comprises a tyrosine kinase inhibitor (TKI). A tyrosine kinase catalyzes the transfer of a phosphate group to the tyrosine residue of a specific protein. If a TKI inhibits an action of a kinase necessary for growth, differentiation or division of a cancer cell, expansion of a cancer cell is slowed. A TKI includes any agent that inhibits the action of one or more tyrosine kinases in a specific or nonspecific fashion. TKIs include small molecules, antibodies, peptides, or anything that directly, indirectly, allosterically, or in any other way inhibits tyrosine residue phosphorylation.

Specific examples of tyrosine kinase inhibitors include N-(trifluoromethylphenyl)-5-methylisoxazol-4-carboxamide, 3-[(2,4-dimethylpyrrol-5-yl)methylidenyl)indolin-2-one, 17-(allylamino)-17-demethoxygeldanamycin, 4-(3-chloro-4-fluorophenylamino)-7-methoxy-6-[3-(4-morpholinyl)propoxyl]quinazoline, N-(3-ethynylphenyl)-6,7-bis(2-methoxyethoxy)-4-quinazolinamine, BIBX1382, 2,3,9,10,11,12-hexahydro.10-(hydroxymethyl)-10-hydroxy-9-methyl-9,12-epoxy-1H-diindolo[1,2,3-fug:3',2', 1'-k 1]pyrrolo[3,4-i][1,6]benzodiazocin-1-one, SH268, genistein, STI571, CEP2563, 4-(3-chlorophenylamino)-5,6-dimethyl-7H-pyrrolo [2,3-d]pyrimidinemethane sulfonate, 4-(3-bromo-4-hydroxyphenyl)amino-6,7-dimethoxyquinazoline, 4-(4'-hydroxyphenyl)amino-6,7-dimethoxyquinazoline, SU6668, STI571A, N-4-chlorophenyl-4-(4-pyridylmethyl)-1-phthalazinamine, N-[2-(diethylamino)ethyl]-5-[(Z)-(5-fluoro-1,2-dihydro-2-oxo-3H-indol-3-ylidine)methyl]-2,4-dimethyl-1H-pyrrole-3-carboxamide (sunitinib), 4-[4-[[4-chloro-3 (trifluoromethyl)phenyl]carbamoylamino] phenoxy]-N-methyl-pyridine-2-carboxamide (sorafenib), and EMD121974.

In another embodiment, a tyrosine kinase inhibitor has activity upon Vascular Endothelial Growth Factor Receptor 2 (VEGFR2) and Vascular Endothelial Growth Factor Receptor 3 (VEGFR3). VEGFR2 and VEGFR3 are tyrosine kinases that phosphorylate proteins necessary for angiogenesis. Tyrosine kinase inhibitor may inhibit either VEGFR2 or VEGFR3 singly, both VEGFR2 and VEGFR3 to the exclusion of all other targets, or VEGFR2 and/or VEGFR3 in combination with one or more additional tyrosine kinases or other targets.. Tyrosine kinase inhibitors that inhibit VEGFR2 and/or VEGFR3 include sunitinib and sorafenib, but could be any inhibitor that targets VEGFR2 singly or in combination with any other tyrosine kinase, any inhibitor that targets VEGFR3 singly or in combination with any other tyrosine kinase, or any inhibitor that targets either VEGFR2 and VEGFR3 to the exclusion of or in combination with any other tyrosine kinase.

As used herein, VEGFR2 has the sequence provided below.

gcagcatcgg acaagacccc cagcacttgg gggttcaggc ccggcagggc gggcagaggg
ctggaggccc aggctgggaa ctcatctggt tgaactctgg tggcacagga gtgtcctctt
ccctctctgc agacttccca gctaggaaga gcaggactcc aggcccaagg ctcccggaat
tccgtcacca cgactggcca gggccacgct ccagctgccc cggcccctcc ccctgagatt
cagatgtcat ttagttcagc atccgcaggt gctggtcccg gggccagcac ttccatggga
atgtctcttt ggcgacctcc tttcatcaca ctgggtggtg gcctggtccc tgttttccca
cgaggaatct gtgggtctgg gagtcacaca gtgttggagg ttaaggcata cgagagcaga
ggtctcccaa acgccctttc ctcctcaggc acacagctac tctccccacg agggctggct
ggcctcaccc acccctgcac agttgaaggg aggggctgtg tttccatctc aaagaaggca
tttgcagggt cctcttctgg gcctgaccaa acagccaact agcccctggg gtggccacca
gtatgacagt attatacgct ggcaacacag aggcagcccg cacacctgcg cctgggtgtt
gagagccatc ctgcaagtct ttttcaacag aacttcacag actgttagag ctgctgagaa
gaatttgctt tccgaattca gcctggaagg cgcccaggga cagctgtact gagtctagat
gactctgacc cccgccccag gtcaaggcca gcagagcagt cagtgcctct ggagaaggcc
cttgctctcc cacctggccc agactccgag gagcctgggt ctggagctgc cggtctggtt
cttcccttta gagcccggat ctgccacctg cggcccctcc caagccgtga accagctcat
gagagatgaa cactgtggga tccactcagg aaggctcggg gctggcacaa aggaccaccc
agcattgccc tgtgccaccc agcactcagt ggacattctg gggacctgcc ttcagccttt
tcctgccctg tgcctgacat cagcaccctg gctggtcaga atgccgccct cccagaggag
cagccgagag atcccctgaa ggctggaggc attctgctca ggacccctat cccagctcac
agtgcccaac catctcacca ggagaaagag ccacatcccc acgttaggac cacggagact
gaccaccacc ctgacccccc aaacccacgc accagacgct tgcaggacag gcgccgcgca

gcgggcaggg gcttgcccgg ccgaccctcc cctccccacc tcccccactg cgcgttactc
caggatatgc cgagtgcacg tataaggtca tcttcgtcgt ccccgtggac ctcccccttc
ctctgcacgt cgtccaacgt gggactggcg tgtcaggctt ccctgggagg atctggaggt
tgttctctgc agagaaccag cctggctcct ggcgcgcacc tctgctccct tctcctcact
acccacccac gcatgtaccg ggaaaaaaac tactatgccc ttctagacca tgttctgaga
aaagatcgaa aatatttaac aagagataat aataaatctg atgccggtct ttgtgtgtgt
tgcgga (SEQ ID NO:2)

VEGFR3 has the sequence provided below.

gcagcatcgg acaagacccc cagcacttgg gggttcaggc ccggcagggc gggcagaggg
ctggaggccc aggctgggaa ctcatctggt tgaactctgg tggcacagga gtgtcctctt
ccctctctgc agacttccca gctaggaaga gcaggactcc aggcccaagg ctcccggaat
tccgtcacca cgactggcca gggccacgct ccagctgccc cggcccctcc ccctgagatt
cagatgtcat ttagttcagc atccgcaggt gctggtcccg gggccagcac ttccatggga
atgtctcttt ggcgacctcc tttcatcaca ctgggtggtg gcctggtccc tgttttccca
cgaggaatct gtgggtctgg gagtcacaca gtgttggagg ttaaggcata cgagagcaga
ggtctcccaa acgccctttc ctcctcaggc acacagctac tctccccacg agggctggct
ggcctcaccc acccctgcac agttgaaggg aggggctgtg tttccatctc aaagaaggca
tttgcagggt cctcttctgg gcctgaccaa acagccaact agcccctggg gtggccacca
gtatgacagt attatacgct ggcaacacag aggcagcccg cacacctgcg cctgggtgtt
gagagccatc ctgcaagtct ttttcaacag aacttcacag actgttagag ctgctgagaa
gaatttgctt tccgaattca gcctggaagg cgcccaggga cagctgtact gagtctagat
gactctgacc cccgccccag gtcaaggcca gcagagcagt cagtgcctct ggagaaggcc
cttgctctcc cacctggccc agactccgag gagcctgggt ctggagctgc cggtctggtt
cttcccttta gagcccggat ctgccacctg cggcccctcc caagccgtga accagctcat
gagagatgaa cactgtggga tccactcagg aaggctcggg gctggcacaa aggaccaccc
agcattgccc tgtgccaccc agcactcagt ggacattctg gggacctgcc ttcagccttt
tcctgccctg tgcctgacat cagcaccctg gctggtcaga atgccgccct cccagaggag
cagccgagag atcccctgaa ggctggaggc attctgctca ggacccctat cccagctcac
agtgcccaac catctcacca ggagaaagag ccacatcccc acgttaggac cacggagact
gaccaccacc ctgacccccc aaacccacgc accagacgct tgcaggacag gcgccgcgca
gcgggcaggg gcttgcccgg ccgaccctcc cctccccacc tcccccactg cgcgttactc
caggatatgc cgagtgcacg tataaggtca tcttcgtcgt ccccgtggac ctcccccttc
ctctgcacgt cgtccaacgt gggactggcg tgtcaggctt ccctgggagg atctggaggt
tgttctctgc agagaaccag cctggctcct ggcgcgcacc tctgctccct tctcctcact
acccacccac gcatgtaccg ggaaaaaaac tactatgccc ttctagacca tgttctgaga
aaagatcgaa aatatttaac aagagataat aataaatctg atgccggtct ttgtgtgtgt
tgcgga (SEQ ID NO:3)

However, contemplated is any sequence identifiable as VEGFR2 or VEGFR3 on the basis of its ability to be inhibited by one or more tyrosine kinase inhibitors, its ability to recognize a specific ligand, or its ability to perpetuate an intracellular signal. A sequence may display any one of these characteristics and any combination thereof. A sequence includes any mutation, truncation, or addition of extra nucleotides. A sequence having at least 60% 70%, 80%, or 90% identity to SEQ ID NO. 2 in the case of VEGFR2 and at least 60% 70%, 80%, or 90% identity to SEQ ID NO. 3 in the case of VEGFR3 at the genomic, mRNA, or protein level.

MicroRNA's (miR's) are non-coding RNAs having 18 to 36 nucleotides, in one embodiment 21 to 25 nucleotides in length that inhibit gene expression by binding to a sequence complementary to the miR sequence, often located in the 3' untranslated region (UTR) of the target mRNA. Mechanisms of gene silencing include repression of protein translation and downregulation of protein expression.

As used herein, miR-497 has the sequence provided below.
cagcagcaca cugugguuug u (SEQ ID NO:1)

However, contemplated is any sequence identifiable as miR-497 including mutations, truncations, or additions of one or more nucleotides that is capable of binding the 3' UTR of VEGFR2 or VEGFR3 with such binding resulting in a reduced VEGFR2 or VEGFR3 expression. A sequence having at least 60%, 70%, 80%, or 90% identity to SEQ ID NO.1 at the genomic or mRNA level. The concept of miR-497 includes one or more non-nucleotide small molecule compositions of matter derived from miR-497 capable of specifically binding to the 3' UTR of VEGFR2/3 such that VEGFR2/3 expression is silenced.

While a specific target is identified by a nucleic acid sequence, such as a cDNA, mRNA or protein sequence, a specific target is not limited to the products of that exact sequence. Rather, a specific target identified by a nucleic acid sequence encompasses all sequences that, when their expression is assessed, yield positive expression when assessed by the same method as the specific target.: In one embodiment, if expression of a specific target in a sample is assessed by immunohistochemical analysis, and if a sample expresses a sequence different from the sequence used to identify the specific target (*e.g*., a variation of one or more nucleic acid molecules,) but positive expression is still determined, then the specific target encompasses the sequence expressed by the sample.

Expression encompasses all processes through which material derived from a nucleic acid template is produced. Expression thus includes RNA transcription, mRNA splicing, protein translation, protein folding, post-translational modification, membrane transport, associations with other molecules, addition of carbohydrate moeties to proteins, phosphorylation, protein complex formation and any other process along a continuum that results in biological material derived from genetic material. Expression also encompasses all processes through which the production of material derived from a nucleic acid template is actively or passively suppressed. Such processes include all aspects of transcriptional and translational regulation. Examples include heterochromatic silencing, transcription factor inhibition, any form of RNAi silencing, microRNA silencing, alternative splicing, protease digestion, post-translational modification, and alternative protein folding.

Expression is assessed by any number of methods used to detect material derived from a nucleic acid template used currently in the art and yet to be developed. Examples of such methods include any nucleic acid detection method including, but not limited to, microarray analysis, RNA *in situ* hybridization, RNAse protection assay, Northern blot, reverse transcriptase PCR, quantitative PCR, quantitative reverse transcriptase PCR, quantitative real-time reverse transcriptase PCR, or any other method of detecting a specific nucleic acid known or yet to be disclosed. Other examples include any process of detecting expression that uses an antibody including, but not limited to, flow cytometry, immunohistochemical methods, ELISA, Western blot, and immunoaffinity chromatograpy. Antibodies may be monoclonal, polyclonal, or any antibody fragment including an Fab, F(ab)2, Fv, scFv, phage display antibody, peptibody, multispecific ligand, or any other reagent with specific binding to a target. Such methods also include direct methods used to assess protein expression including, but not limited to HPLC, mass spectrometry, protein microarray analysis, PAGE analysis, isoelectric focusing, 2-D gel electrophoresis, and enzymatic assays. Samples from which expression is detected include single cells, whole organs or any fraction of a whole organ, whether *in vitro*, *ex vivo*, *in vivo*, or *post-mortem.*

Other methods used to assess expression include the use of natural or artificial ligands capable of specifically binding a target, including a protein, carbohydrate, fat, nucleic acid, catalytic site, or any combination of these such as an enzyme, glycoprotein, cell membrane, virus, cell, organ, organelle, or any other multimolecular structure that constitutes a target that is specifically bound by a ligand. Such ligands include antibodies, antibody complexes, conjugates, natural ligands, small molecules, nanoparticles, or any other molecular entity capable of specific binding to a target. Ligands are associated with a label such as a radioactive isotope or chelate thereof, dye (fluorescent or nonfluorescent,) stain, enzyme, metal, or any other substance capable of aiding a machine or a human eye from differentiating a cell expressing a target from a cell not expressing a target. Additionally, expression may be assessed by monomeric or multimeric ligands associated with substances capable of killing a cell. Such substances include protein or small molecule toxins, cytokines, pro-apoptotic substances, pore forming substances, radioactive isotopes, or any other substance capable of killing a cell.

Positive expression includes any difference between a cell expressing a specific target and a cell that does not express a specific target. The exact nature of positive expression varies by the method, but is well known to those practicing a particular method. Positive expression is assessed by a detector, an instrument containing a detector, or by aided or unaided human eye. Examples include, but are not limited to, specific staining of cells expressing a target in an IHC slide, binding of RNA from a sample to a microarray and detection by an instrument capable of detecting the binding to said microarray, a high rate of dye incorporation in real-time RTPCR, detection of fluorescence on a cell expressing a target by a flow cytometer, the presence of radiolabeled bands on film in a Northern blot, detection of labeled blocked RNA by RNAse protection assay, cell death measured by apoptotic markers, cell death measured by shrinkage of a tumor, or any other method by which expression is observed known or yet to be disclosed.

Reduced expression constitutes a lack of positive expression such that there is not a significant difference between a cell expressing a particular target and a cell not expressing the particular target. The concept of reduced expression further encompasses insufficient expression to reach or exceed a threshold, cutoff, or level that results in a particular cellular or physiological response. For example, reduced expression includes the expression of a particular target in a test cell that is positive expression relative to a control cell known not to express the target. However, because the expression of the target in the test cell is insufficient to cause a particular physiological response (*e.g*., rendering the cell sensitive to a particular drug), the expression in the test cell is still classified as reduced expression. Similarly, the concept of positive expression also encompasses expression sufficient to cause a physiological response.

Also provided are methods of assessing the expression of a target in any biological sample from which the expression is assessed. One skilled in the art knows how to select a particular biological sample and how to collect said sample depending upon whether or not expression of germline DNA, tumor DNA, mRNA, or any form of protein is assessed. Examples of sources of samples include, but are not limited to, biopsy or other *in vivo* or *ex vivo* analysis of prostate, breast, skin, muscle, facia, brain, endometrium, lung, head and neck, pancreas, small intestine, blood, liver, testes, ovaries, colon, skin, stomach, esophagus, spleen, lymph node, bone marrow, kidney, placenta, or fetus tissues. In one embodiment, a sample comprises a fluid sample, such as peripheral blood, lymph fluid, ascites, serous fluid, pleural effusion, sputum, cerebrospinal fluid, amniotic fluid, lacrimal fluid, stool, or urine. In another embodiment, a sample comprises primary or metastatic NSCLC cells. In yet another embodiment, a sample comprises blood. MicroRNA is readily detectable in blood and blood compartments such as serum or plasma by a number of methods. (Chen X et al, Cell Research 18 983-984, October 2008).

### EXAMPLES

### Example 1.

Expression of a microRNA (miR) with specificity to the 3'UTR of VEGFR2 or VEGFR3 is capable of binding the UTR and silencing the VEGFR2 or VEGFR3 (VEGFR 2/3) expression. Positive expression of such miR indicates reduced VEGFR2/3 expression. Tumors with reduced VEGFR2/3 expression are resistant to VEGFR 2/3 specific tyrosine kinase inhibitors. Conversely, if a tumor displays reduced expression of a miR capable of downregulating VEGFR-2/3, then the result is a more robust VEGFR-2/3 expression, indicating that the tumor is more sensitive to VEGFR2/3 specific tyrosine kinase inhibitors. Assessing VEGFR2/3 expression by miR has advantages over assessing VEGFR2/3 protein directly. Expression by miR is assessed quickly by PCR and high throughput sequencing methods. Further, miR is available in blood and the expression of an individual miR is easily assessed in plasma, serum, or other blood fractions. Such assays allow easy presymptomatic surveillance of a number of diseases, especially cancer.

Search of a publicly available database revealed that miRs, based on their sequence, potentially regulate VEGFR-2/3 expression (Targetscan Database, Whitehead Institute for Biomedical Research, 2006-2008). This comprised searching the 3' UTR of both genes for potential miR binding sites. One core predicted binding site GCTGCT was common for both the VEGFR2 and VEGFR3 3'UTR's. Using seed sequences, miR-497 was identified as possibly capable of regulating VEGFR2/3. MiR-497 is located on chromosome 17p. The fact that allelic loss in chromosome 17p is frequent in lung cancer (Tonon et al., Proc Natl Acad Sci USA 102:9625-9630, 2005) further led to the selection of miR-497. Allelic loss of 17p occurs at a 50% rate in lung cancer. Additionally, chromosome 17p is situated in close proximity (<1 MB) to the TP53 gene locus, a frequent site of loss of heterozygosity in cancer generally (Chmara et al., Anticancer Res 24:4259-4263, 2004).

MiR-497 expression, VEGFR2/VEGFR3 protein and mRNA expression, and sensitivity of specific tyrosine kinase inhibitor sunitinib to the VEGFR2/3 were assessed in six cell lines derived from non-small cell lung cancer (H1703, A549, H520, H322C, H358, and H157) and are summarized in Table 1 (below).

**Table 1**

| Data | H1703 (Adenocarcinoma) | A549 (Adenocarclnoma) | H520 (Squamous Cell Carcinoma) | H322C (Bronchioloalveolar Carcinoma) | H358 (Bronchloloalveolar Carcinoma) | H157 (Squamous Cell Carcinoma) |
|---|---|---|---|---|---|---|
| microRNA-497 expression | High | High | High | Low | Low | Low |
| VEGFR2 mRNA level (I/M) | Up/Up | NC/Down | Up/Up | Down/Up | NC/Up | NC/Up |
| VEGFR3 mRNA level (1/M) | NC/Up | Down/Down | Up/NC | Down/Up | Up/Down | NC/Up |
| I/M = Inhibitor/Mimic, NC = no change from control | | | | | | |

Positive expression of miR-497 is seen in the H1703, A549, and H520 cell_lines, while reduced expression is seen in H322C, H358, and H157. The table further shows changes in VEGFR2 and VEGFR3 mRNA expression after transfection with miR-497 inhibitor(I) or miR-497 mimic (M) (an RNA with a similar sequence to that of miR-497) normalized to the miR-497 expression with a control miR in which all three had been normalized to act in mRNA expression.

Transfection of miR-497 mimic and inhibitor (an RNA with a sequence similar to the antisense sequence of miR-497) into the indicated cell lines was performed, followed by Western blotting and qRT-PCR. Densitometry measurements were taken from Western blot images using Scion Image.

Table 2 summarizes the expression of VEGFR2 protein and VEGFR3 protein in the six listed above cell lines as the IC₅₀ of each cell line to sunitinib (below).

**Table 2**

| Data | H1703 | H520 | H322C | H358 | A549 | H157 |
|---|---|---|---|---|---|---|
| Histology | Adenocarcinoma | Squamous Cell | Bronchioloalveolar | Bronchioloalveolar | Adenocarcinoma | Squamous Cell |
| VEGFR2 protein | Present | Absent | Present | Present | Present | Present |
| VEGFR3 protein | Absent | Absent | Present | Present | Present | Present |
| IC₅₀ | <0.5 µM | 1-3 µM | 9-10 µM | >10µM | >10µM | >10 µM |

The first two rows of Table 2 summarize the expression of VEGFR2 protein and VEGFR3 protein in the six listed cell lines by Western blot. The term "present" indicates positive expression of VEGFR2 or VEGFR3 protein.

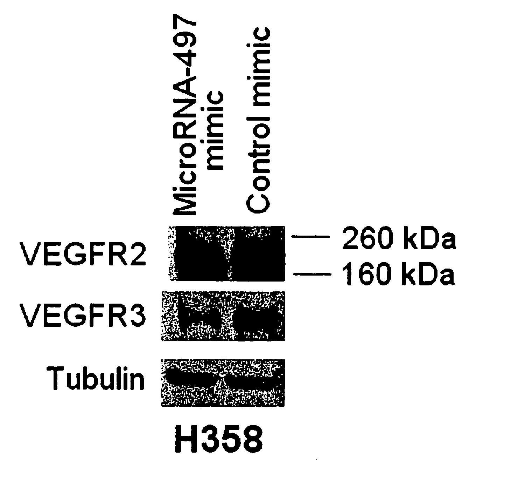

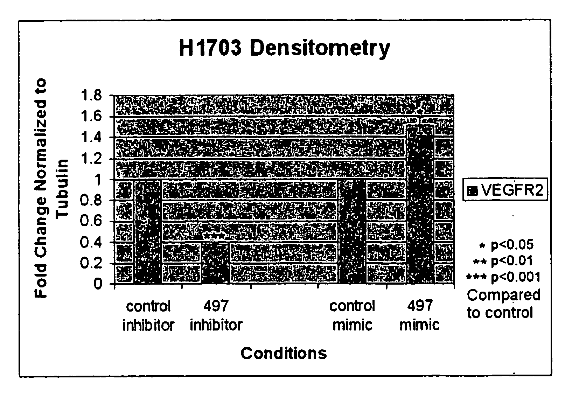

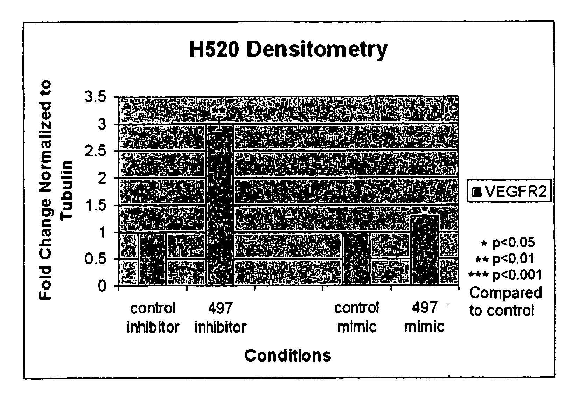

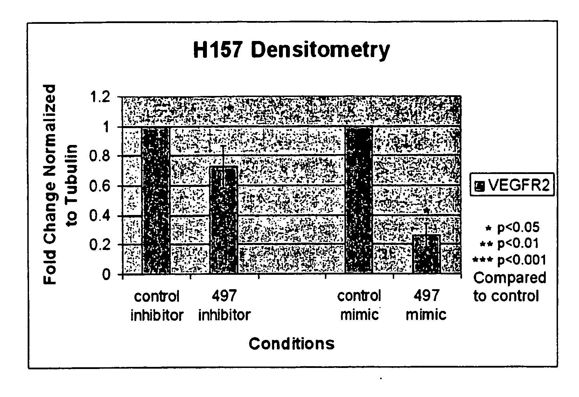

As shown in Figures 1 to 5, reduced expression of the VEGFR2 protein with transfection of miR-497 mimic was observed in four of the six tested cell lines. Reduced expression of the VEGFR2 mRNA was not observed in any of the tested cell lines transfected with miR-497 mimic. Increased expression of the VEGFR2 protein was observed in three of the six lines transfected with miR-497 inhibitor. Increased expression of the VEGFR2 mRNA was observed in two of the six cell lines transfected with the miR-497 inhibitor.

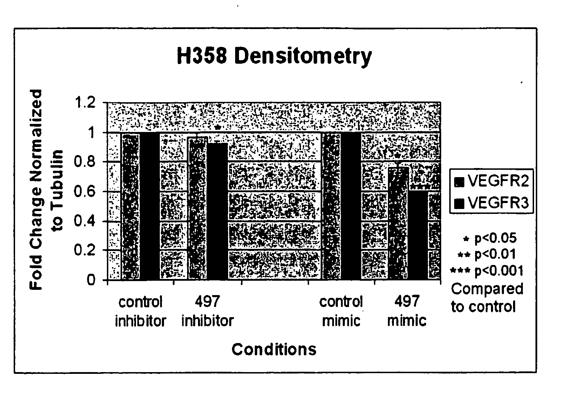

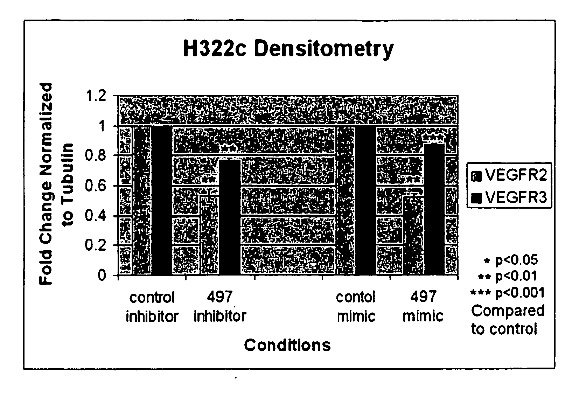

As shown in Figures 2 and 7, reduced expression of the VEGFR3 protein was observed with transfection of miR-497 mimic in one of the two cell lines in which it was assessed. Reduced expression of the VEGFR3 message was observed with transfection of miR-497 mimic in one of the six tested cell lines. Increased expression of the VEGFR3 protein was not observed in either of the cell lines transfected with the miR-497 inhibitor in which the VEGFR3 protein expression was assessed. Increased VEGFR3 message was observed in two of the six cell lines when those lines were transfected with the miR-497 inhibitor.

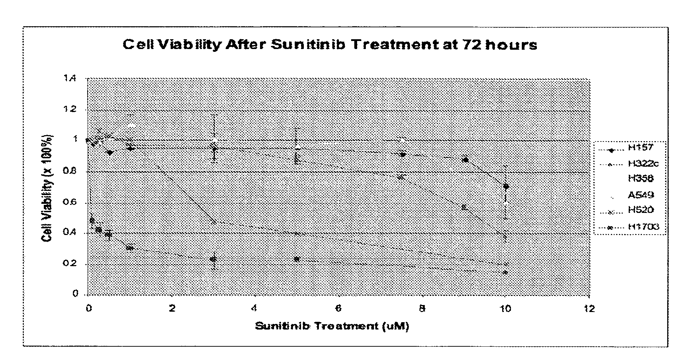

As shown in Table 2 and Figure 8, untransfected cell lines H 1703 and H520 were sensitive to sunitinib, displayed positive expression of miR-497 and both lacked the VEGFR3 protein expression. Additionally, H520 lacked the VEGFR2 protein expression. Untransfected cell lines A549, H322C, H358, and H157 were resistant to sunitinb and displayed positive expression of both VEGFR2 and VEGFR3. Of these, only A549 displayed positive expression of miR-497. In general, *in vitro* exposure increased sensitivity to VEGFR-2/3 sunitinib correlated with a positive expression of microRNA-497.

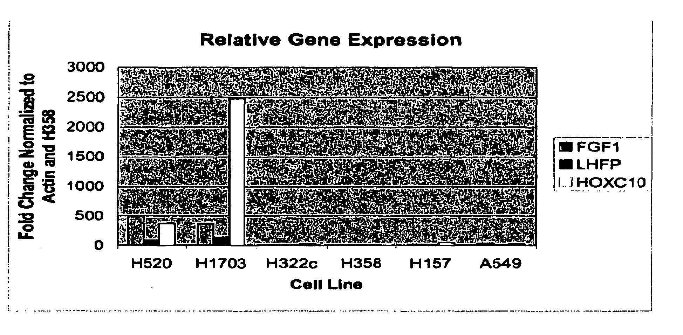

As shown in Figure 9, microarray gene expression data from NCBI's GEO GSE 4342 were normalized by 'per chip normalization' and 'per gene normalization' using GeneSpring between resistant and sensitive NSCLC lines. Genes that were identified as having statistically significant differences (p<0.01) when grouped as sunitinib resistant (defined solely for the purposes of this example as having IC50 > 9 µM) and sunitinib sensitive (defined solely for the purposes of this example as having an IC50 < 3 µM), were validated by qRT-PCR. Genes meeting those criteria were confirmed by RTPCR. Using qRT-PCR, it was revealed that FGF1 (SEQ ID NO:4), HOXC10 (SEQ ID NO:5), and LHFP (SEQ ID NO:6) were present in NSCLC lines and were resistant to sunitinib. Surprisingly, pathway analysis revealed that these 3 genes were not part of canonical pathways of resistance to sunitinib.

Sequences corresponding to SEQ ID NO:4, SEQ ID NO:5 and SEQ ID NO:6 are provided below.

FGF1

agctgcagta gcctggaggt tcagagagcc gggctactct gagaagaaga caccaagtgg
attctgcttc ccctgggaca gcactgagcg agtgtggaga gaggtacagc cctcggccta
caagctcttt agtcttgaaa gcgccacaag cagcagctgc tgagccatgg ctgaagggga
aatcaccacc ttcacagccc tgaccgagaa gtttaatctg cctccaggga attacaagaa
gcccaaactc ctctactgta gcaacggggg ccacttcctg aggatccttc cggatggcac
agtggatggg acaagggaca ggagcgacca gcacattcag ctgcagctca gtgcggaaag
cgtgggggag gtgtatataa agagtaccga gactggccag tacttggcca tggacaccga
cgggctttta tacggctcac agacaccaaa tgaggaatgt ttgttcctgg aaaggctgga
ggagaaccat tacaacacct atatatccaa gaagcatgca gagaagaatt ggtttgttgg
cctcaagaag aatgggagct gcaaacgcgg tcctcggact cactatggcc agaaagcaat
cttgtttctc cccctgccag tctcttctga ttaaagagat ctgttctggg tgttgaccac

tccagagaag tttcgagggg tcctcacctg gttgacccaa aaatgttccc ttgaccattg
gctgcgctaa cccccagccc acagagcctg aatttgtaag caacttgctt ctaaatgccc
agttcacttc tttgcagagc cttttacccc tgcacagttt agaacagagg gaccaaattg
cttctaggag tcaactggct ggccagtctg ggtctgggtt tggatctcca attgcctctt
gcaggctgag tccctccatg caaaagtggg gctaaatgaa gtgtgttaag gggtcggcta
agtgggacat tagtaactgc acactatttc cctctactga gtaaacccta tctgtgattc
ccccaaacat ctggcatggc tcccttttgt ccttcctgtg ccctgcaaat attagcaaag
aagcttcatg ccaggttagg aaggcagcat tccatgacca gaaacaggga caaagaaatc
cccccttcag aacagaggca tttaaaatgg aaaagagaga ttggattttg gtgggtaact
tagaaggatg gcatctccat gtagaataaa tgaagaaagg gaggcccagc cgcaggaagg
cagaataaat ccttgggagt cattaccacg ccttgacctt cccaaggtta ctcagcagca
gagagccctg ggtgacttca ggtggagagc actagaagtg gtttcctgat aacaagcaag
gatatcagag ctgggaaatt catgtggatc tggggactga gtgtgggagt gcagagaaag
aaagggaaac tggctgaggg gataccataa aaagaggatg atttcagaag gagaaggaaa
aagaaagtaa tgccacacat tgtgcttggc ccctggtaag cagaggcttt ggggtcctag
cccagtgctt ctccaacact gaagtgcttg cagatcatct ggggacctgg tttgaatgga
gattctgatt cagtgggttg ggggcagagt ttctgcagtt ccatcaggtc ccccccaggt
gcaggtgctg acaatactgc tgccttaccc gccatacatt aaggagcagg gtcctggtcc
taaagagtta ttcaaatgaa ggtggttcga cgccccgaac ctcacctgac ctcaactaac
ccttaaaaat gcacacctca tgagtctacc tgagcattca ggcagcactg acaatagtta
tgcctgtact aaggagcatg attttaagag gctttggccc aatgcctata aaatgcccat
ttcgaagata tacaaaaaca tacttcaaaa atgttaaacc cttaccaaca gcttttccca
ggagaccatt tgtattacca ttacttgtat aaatacactt cctgcttaaa cttgacccag
gtggctagca aattagaaac accattcatc tctaacatat gatactgatg ccatgtaaag
gcctttaata agtcattgaa atttactgtg agactgtatg ttttaattgc atttaaaaat
atatagcttg aaagcagtta aactgattag tattcaggca ctgagaatga tagtaatagg
atacaatgta taagctactc acttatctga tacttattta cctataaaat gagattttg
ttttccactg tgctattaca aattttcttt tgaaagtagg aactcttaag caatggtaat
tgtgaataaa aattgatgag agtgttagct cctgtttcat atgaaattga agtaattgtt
aactaaaaac aattccttag taactgaact gtcatattta gaatggaagg aaaatgacag
tttgtgaaag ttcaaagcaa tagtgcaatt gaagaattga cctaagtaag ctgacattat
ggttaataat agtattttag atttgtgcag caaaataatt tcataacttt tttgtttttg
ttacttggat aagatcaatc tgttttattt tagtaaatct ttgcaggcaa gttagagaaa
atgcagtgtg gcttaacgtc tctttagtat gaagatttgg ccagaaaaag atacccagag
aggaaatcta agataattat aatggtccat acttttattt gtatgaatca aactcaagca
taacattggc caaggaaaat taaataccat tgctaacttg tgaaatggaa gtctgtgatt
tcggagatgc aaagcattgt agtaaaaaca ccaatgtgac ctcgaccatc tcagcccaga
tatcattcat atatctgttc aatgactatt aaggtgccta ctgtgtgcta ggcactgtac

tggatactgg ggaccttgtc tgtctggttt gctgctgtat cttctcccag ggcattatat
ttatgatgaa agatgctgtg gattcaattc tttcagtcaa gaataaacac agactttgta
ggttcctgct gaataaagca aatcccagaa acccagattt tggaagaatc agcaacccca
gcataaaata aacccctatc aaaatgtcag aggacatggc aaggtaaact tagcattttc
aactttagaa ccgggtcagc ttcaggggga ctgctttcaa atcagccaaa gagcctgtca
gatcttctta gaaggaagag gttggtagtt ccctgctctg ttttgaacat gctctagttt
attaacctgg ggacattccc attgctgtct taagtaagtc tcatagccag ctcctgtcac
gtgactctca tatggattca ttttcgggcc agctctgaac aaagcatcat gaacatatgt
gcttttggtc gtttgcaatg tgatggtggt ggaggtaggt attggtttcc ttggaaggca
tgataagaaa gattcacaat ggccaacagt gtgtatgaac aaaaaactga ttggagcatc
agctagtact gaaggtcctt gctttgtgtc agaggcaaag gaacccaagg cgccaagtcc
tcagccttga gtgtactgct gacaactaaa ctcacaggct gcaaagcaga cctctgatga
agatgcctgt tatttcacat cactgtcttt ttgtgtatca tagtctgcac cttacaaata
ttaataaatg ttccaataat aggtgaaaaa aaaaa (SEQ ID NO:4)

HOXC10

cctcccctcc aaccgcgccc cccctcccgg atggggaaaa aaaaagatgt cagctcctcc
gctgtagtat tgctccttaa aaacccctct ctctgaaaat gacatgccct cgcaatgtaa
ctccgaactc gtacgcggag cccttggctg cgcccggcgg aggagagcgc tatagccgga
gcgcaggcat gtatatgcag tctgggagtg acttcaattg cggggtgatg aggggctgcg
ggctcgcgcc ctcgctctcc aagagggacg agggcagcag ccccagcctc gccctcaaca
cctatccgtc ctacctctcg cagctggact cctggggcga ccccaaagcc gcctatcgcc
tggaacaacc tgttggcagg ccgctgtcct cctgctccta cccacctagt gtcaaggagg
agaatgtctg ctgcatgtac agcgcagaga agcgggcgaa aagtggcccc gaggcagctc
tctactccca ccccttgccg gagtcctgcc ttggggagca cgaggtaccc gtgcccagct
actaccgcgc cagcccgagc tactccgcgc tggacaagac gccccactgt tctgggcca
acgacttcga agcccctttc gagcagcggg ccagtctcaa cccgcgcgcc gaacatctgg
aatcgcctca gctggggggc aaagtgagtt tccctgagac ccccaagtcc gacagccaga
cccccagccc caatgaaatc aagacggagc agagcctggc gggccctaaa gggagcccct
cggagagcga aaaggagagg gccaaagctg ccgactccag cccagacacc tcggataacg
aagcgaaaga ggagataaag gcagaaaaca ccacaggaaa ttggctgaca gcaaagagcg
gaaggaagaa gaggtgcccc tatactaaac accagacgct ggaattggag aaagaatttc
tgttcaatat gtatttgacg cgagagcgcc gcctggagat tagcaagacc attaacctta
cagacagaca agtcaaaatc tggtttcaaa atcgcagaat gaaactcaag aaaatgaacc
gagagaatcg gatccgggaa ctgacctcca attttaattt cacctgagag cgcggcctct
cctcctccct tcccgctcct tcctctcccc gcccctcctc cctttgtgcc tggtgatata
tttttttttc ctccctgagt ataaatgcaa tgcgactgca aaaaaggcaa agacctcaga
ctctccttcc aagggacctg tggttcgtgc tgcgaagatg cttccactta aagcatgaga

aatggggtgc cgggatgtgg ggtgtggtgt gtgccctcat agatgggggt gggagtgtgg
ctggtgtgtg tgtcaagccc tcactcaccc acgcactcac acacagcatt ctgttctcca
tgcaaagtta agatcgaatc catccgcttg taggggaaaa aaaggaaaaa aattaaccag
agagggtctg taatctcgca gagcacaggc agaatcgttc cttccttgct gcatttcctc
cttagactaa tagacgtttt ggaaagttcg gctagtgttc gtgtgtttgt cgtagcaccc
agagcctcca ccaaaccctc tccatgtctt tacctcccag tcgctctaag aatctgcttg
aagtctcgta tttgtactgc tttctgcttt tctcccaccc ctcctagcac ccccacatcc
cccatctagt aacatctcag aaatttcatc cagaggaaca aaaaaattaa aaatagaaca
tagcaaagca aagacagaat gccccccccc ccaaatattg tcctgtccct gtctgggagt
tgtgttattt aaagatattc tgtatgttgt atcttttgca tgtagcttcc ttaatggaga
aaaaaaaacc taataaattt ccagaatcat aatcctcaaa aaaaaaaaaa aaaaaaaaaa
aaaaaaaaaa aaaaaaaaaa aaaaaaaaaa aaaaaaa (SEQ ID NO:5)

LHFP

aggaggcggt gcgtgcctcg cctgccaaag ggagatccgc tcctctgcgt gcgatccccg
gcgcccgcgc gcgcccacag cgctccgcca gagctgccgc cgcggactcg ccgggagtgg
gggtctccgc tggtgccagc ccgcttctgg agaccctccg cctcctgcca acccctgctc
ttccaggtcg ggccccgggg ttctgcggct gttagggaca gaggcaaaga agggcaggac
ggtccggttt cccgtggatg ttcccgcccg agaaagacag caagttgtgt gtgcgcccgg
gacgcgggag ggaaggtagc cgccgcccgc cagccatgga ccatcatctt tagtgcagag
gatggaaagt tgatgcccag taagactgaa gatccattct gcattacgga actgtggatt
atctgtgggt ccctggtgat ttcacacctt cattcactcc tgcagtccct gaacacttac
ttggggtcct cattgcccta tctggtgaaa gatggcatcc agcctgactt gtactggagt
aatctgggct ttgctgtctt ttctttgtgc tgccacctcc tgcgtggggt tctttatgcc
ttactggctc tggggatcac agctgggcaa gcctgtgtcc ttcggtacct tccggaggtg
ctcatatcct gtgcatgatg agagtcggca gatgatggtg atggtggagg aatgtgggcg
ctatgcctcc ttccagggca tccccagcgc agaatggagg atctgcacca tagtgaccgg
cctgggttgt ggcctcctcc tcctggtggc gctcactgcc ctcatgggtt gctgtgtttc
cgacctcatc tccaggacag tgggaagagt ggctggagga attcagtttc ttgggggctt
gttgattggt gctggctgtg ccctctaccc cttgggctgg gacagtgagg aagtccggca
gacttgtggc tacacttctg gccagtttga cctggggaag tgtgaaatcg gctgggccta
ctactgcacg ggagcaggtg ccactgccgc catgctgctg tgcacgtggc tggcttgctt
ttcgggcaag aaacagaagc actacccata ctgagatgga gctaccaaga gcagacagag
gagaagatgg gccaaagggg cttggagagg tcaaaacatc cacctacctt caaaaggtgg
gatagtagtt ctaatccaat acaatgctaa taaaatgaaa cccgataaaa tcaggaacat
gatataggaa ggaaggattg taggagattt gtgggggaaa aaaaaggaga gtatagaatg
atggagaaaa atggaccaaa ggctaaaaat attgcagggc atcgggtgtt tctattccac
agagtattgt taatgtacaa cacacacaca cacacacaca cacacacaca cacacacaca

cacacaacaa atctacatat acaaacaagg gtttgggttt tagttttttt tttttaaggt
gaggactcag aaaatcaaag ggctagtaga aacagtgtta tgttgggaag cagggtaccc
ccaaagatgt tccctgtagg tcacggcact cccaaaagca cacaagcaca tacagacata
tgcatcccca cacacgccta tgcacaaacg tggattatcg cacagactgg gaggtttagt
ggtgcatttc tcctctgttt tctttttaat atacatttaa aatacagtat tatcacttta
taaaacatac attaagccta ataaatggac caataagcca aactatcagt attttgtata
tcctgcataa actctaattt agttcctcaa catattttca gtgtttatgc agacctttag
agttaagcct ttgtatttcc atgttattcc acaatatgca atatttctct gagtagcttc
tgctatgata ttcttatgaa gaaaaggggc aactttctgt ccactatagg agagaattca
ccattattgt actgtgctgt accacattta tttctatatt cattttgtaa aaaatttaaa
agtgctattt tgtttgtatt tgaaaatctc tgtgaataaa ttctctcttt gatcaataaa
aaaaaaaaaa aaaaaaaaaa (SEQ ID NO:6)

## Claims

1. A method of evaluating sensitivity of a non-small cell lung cancer cell to a tyrosine kinase inhibitor comprising:
assessing an expression of miR-497 (SEQ ID NO:1) in a sample comprising a gene product; and
correlating an increased expression of miR-497 (SEQ ID NO:1) with the sensitivity to the tyrosine kinase inhibitor,
wherein the tyrosine kinase inhibitor inhibits VEGFR2 (SEQ ID NO:2) or VEGFR3 (SEQ ID NO:3).

2. The method of claim 1, wherein the sample comprises a tumor biopsy, or optionally blood.

3. The method of claim 1, wherein the gene product comprises mRNA.

4. The method of any one of claim 1 to 3, wherein assessing the expression of miR-497 (SEQ ID NO:1) comprises using RTPCR, or optionally a microarray, or optionally Northern Blot.

5. The method of any one of claim 1 to 4, wherein the tyrosine kinase inhibitor is sunitinib.

6. The method of any one of claims 1, 2, 4 or 5, wherein the gene product comprises protein.

7. The method of any one of claim 1 to 6, wherein assessing the expression of the gene product comprises using a ligand capable of specifically recognizing the gene product,
wherein the ligand comprises an antibody, wherein assessing the expression of the gene product comprises using immunohistochemical methods, or optionally ELISA, or optionally flow cytometry, or optionally mass spectrometry.

8. A method of determining the treatability of a non-small cell lung cancer cell in a subject comprising:
assessing an expression of miR-497 (SEQ ID NO: 1) in a sample;
correlating the increased expression of miR-497 (SEQ ID NO: 1) with the sensitivity to a tyrosine kinases inhibitor; and
determining that the tyrosine kinase inhibitor
wherein the tyrosine kinase inhibitor inhibits VEGFR2 (SEQ ID NO:2) or VEGFR3 (SEQ ID NO:3) is suitable

9. The method of claim 8, wherein the sample is a human sample, or optionally comprises a blood fraction, or optionally a tumor biopsy.

10. The method of claim 9, wherein the cancer cell comprises a loss of heterozygosity in chromosomal region 17p.

11. The method of any one of claim 8 to 10, wherein the tyrosine kinase inhibitor is sunitinib.

## Patentansprüche

1. Ein Verfahren zur Einstufung der Empfindlichkeit eines nicht-kleinzelligen Lungenkarzinoms gegen einen Tyrosinkinase-Inhibitor bestehend aus:
Beurteilung einer Expression von miR-497 (SEQ ID NR: 1) in einer Probe, die ein Genprodukt umfasst; und
Korrelation einer gesteigerten Expression von miR-497 (SEQ ID NR: 1) mit der Empfindlichkeit gegen den Tyrosinkinase-Inhibitor;
wobei der Tyrosinkinase-Inhibitor VEGFR2 (SEQ ID NR: 2) oder VEGFR3 (SEQ ID NR. 3) hemmt.

2. Das Verfahren entsprechend Anspruch 1, wobei die Probe eine Tumorbiopsie oder optionsweise Blut umfasst.

3. Das Verfahren entsprechend Anspruch 1, wobei das Genprodukt mRNA umfasst.

4. Das Verfahren entsprechend einem der Ansprüche 1 bis 3, wobei die Beurteilung der Expression von miR-497 (SEQ ID NR: 1) die Verwendung von RTPCR oder optionsweise eines Mikroarray oder optionsweise von Northern Blot umfasst.

5. Das Verfahren entsprechend einem der Ansprüche 1 bis 4, wobei der Tyrosinkinase-Inhibitor Sunitinib ist.

6. Das Verfahren entsprechend einem der Ansprüche 1, 2, 4 oder 5, wobei das Genprodukt Protein umfasst.

7. Das Verfahren entsprechend einem der Ansprüche 1 bis 6, wobei die Beurteilung der Expression des Genprodukts die Verwendung eines Liganden umfasst, der in der Lage ist, spezifisch das Genprodukt zu erkennen,
wobei der Ligand einen Antikörper umfasst, wobei die Beurteilung des Genprodukts die Verwendung von immunohistochemischen Verfahren oder optionsweise ELISA oder optionsweise Flusszytometrie oder optionsweise Massenspektometrie umfasst.

8. Ein Verfahren zur Bestimmung der Behandelbarkeit eines nicht-kleinzelligen Lungenkarzinoms bei einem Patienten bestehend aus:
Beurteilung einer Expression von miR-497 (SEQ ID NR: 1) in einer Probe,;
Korrelation einer gesteigerten Expression von miR-497 (SEQ ID NR: 1) mit der Empfindlichkeit gegen den Tyrosinkinase-Inhibitor; und
Bestimmen, dass der Tyrosinkinase-Inhibitor geeignet ist,
wobei der Tyrosinkinase-Inhibitor VEGFR2 (SEQ ID NR: 2) oder VEGFR3 (SEQ ID NR. 3) hemmt.

9. Das Verfahren entsprechend Anspruch 8, wobei die Probe eine Humanprobe ist oder optionsweise eine Blutfraktion oder optionsweise eine Tumorbiopsie umfasst.

10. Das Verfahren entsprechend Anspruch 9, wobei das Karzinom einen Verlust von Heterozygosität in der Chromosomenregion 17p aufweist.

11. Das Verfahren entsprechend einem der Ansprüche 8 bis 10, wobei der Tyrosinkinase-Inhibitor Sunitinib ist.

## Revendications

1. Un procédé d'évaluation de la sensibilité d'une cellule de cancer du poumon non à petites cellules à un inhibiteur de la tyrosine kinase comprenant :
l'évaluation d'une expression de miR-497 (SEQ ID NO:1) dans un échantillon contenant un produit génétique, et
la corrélation d'une expression accrue de miR-497 (SEQ ID NO:1) avec la sensibilité à l'inhibiteur de la tyrosine kinase,
où l'inhibiteur de la tyrosine kinase inhibe VEGFR2 (SEQ ID NO:2) ou VEGFR3 (SEQ ID NO:3).

2. Le procédé selon la Revendication 1, où l'échantillon contient une biopsie tumorale, ou éventuellement du sang.

3. Le procédé selon la Revendication I, où le produit génétique contient mARN.

4. Le procédé selon l'une quelconque des Revendications 1 à 3, où l'évaluation de l'expression de miR-497 (SEQ ID NO:1) comprend l'utilisation de RTPCR, ou éventuellement d'une micro-matrice, ou éventuellement d'un buvardage de northern.

5. Le procédé selon l'une quelconque des Revendications 1 à 4, où l'inhibiteur de la tyrosine kinase est sunitinib.

6. Le procédé selon l'une quelconque des Revendications 1, 2, 4 ou 5, où le produit génétique contient une protéine.

7. Le procédé selon l'une quelconque des Revendications 1 à 6, où l'évaluation de l'expression du produit génétique comprend l'utilisation d'un ligand capable de reconnaître spécifiquement le produit génétique,
où le ligand contient un anticorps, où l'évaluation de l'expression du produit génétique comprend l'utilisation de procédés immunohistochimiques, ou éventuellement de ELISA, ou éventuellement d'une cytométrie en flux, ou éventuellement d'une spectrométrie de masse.

8. Un procédé de détermination de la traitabilité d'une cellule de cancer du poumon non à petites cellules chez un sujet comprenant :
l'évaluation d'une expression de miR-497 (SEQ ID NO:1) dans un échantillon,
la corrélation de l'expression accrue de miR-497 (SEQ ID NO:1) avec la sensibilité à un inhibiteur de la tyrosine kinase, et
la détermination que l'inhibiteur de la tyrosine kinase convient
où l'inhibiteur de la tyrosine kinase inhibe VEGFR2 (SEQ ID NO:2) ou VEGFR3 (SEQ ID NO:3).

9. Le procédé selon la Revendication 8, où l'échantillon est un échantillon humain, ou éventuellement contient une fraction sanguine, ou éventuellement une biopsie tumorale.

10. Le procédé selon la Revendication 9, où la cellule cancéreuse comprend une perte d'hérérozygosité dans la zone chromosomique 17p.

11. Le procédé selon l'une quelconque des Revendications 8 à 10, où l'inhibiteur de la tyrosine kinase est sunitinib.
